(19) Europäisches Patentamt
European Patent Office
Office européen des brevets

(11) Veröffentlichungsnummer: **0 325 998 A2**

(12) **EUROPÄISCHE PATENTANMELDUNG**

(21) Anmeldenummer: **89100750.2**

(22) Anmeldetag: **18.01.89**

(51) Int. Cl.4: **C07C 49/24 , C07C 45/68 , C07C 45/63 , C07C 61/40 , C07C 51/00 , C07C 143/68 , C07C 69/743 , C07C 67/00**

(30) Priorität: **27.01.88 DE 3802273**

(43) Veröffentlichungstag der Anmeldung: **02.08.89 Patentblatt 89/31**

(84) Benannte Vertragsstaaten: **BE CH DE ES FR GB LI NL**

(71) Anmelder: **BASF Aktiengesellschaft**
**Carl-Bosch-Strasse 38**
**D-6700 Ludwigshafen(DE)**

(72) Erfinder: **Wild, Jochen, Dr.**
**An der Marlach 7**
**D-6705 Deidesheim(DE)**
Erfinder: **Liese-Sauer, Thomas, Dr.**
**Sigmund-Hirsch-Platz 10**
**D-6940 Weinheim(DE)**
Erfinder: **Goetz, Norbert, Dr.**
**Schoefferstrasse 25**
**D-6520 Worms 1(DE)**
Erfinder: **Theobald, Hans, Dr.**
**Queichstrasse 6**
**D-6703 Limburgerhof(DE)**
Erfinder: **Wolf, Bernd, Dr.**
**Eichenstrasse 11**
**D-6704 Mutterstadt(DE)**

(54) **3,3-Dimethyl-hex-5-en-2-onderivate, Verfahren zu ihrer Herstellung und ihre Verwendung.**

(57) 3,3-Dimethyl-hex-5-en-2-onderivate der allgemeinen Formel (I)

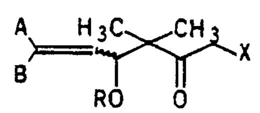

(I)

in der
A Wasserstoff, Halogen oder $C_1$- bis $C_4$-Halogenalkyl
B $C_1$- bis $C_4$-Halogenalkyl
X Wasserstoff oder Halogen und
R Wasserstoff, eine Arylsulfonyl-, Alkylsulfonyl- oder Halogenalkylsulfonylgruppe
bedeuten, Verfahren zur Herstellung der Verbindungen (I) und ihre Verwendung zur Herstellung von Pyrethroiden.

EP 0 325 998 A2

## 3,3-Dimethyl-hex-5-en-2-onderivate, Verfahren zu ihrer Herstellung und ihre Verwendung

Die vorliegende Erfindung betrifft 3,3-Dimethyl-hex-5-en-2-onderivate der allgemeinen Formel (I)

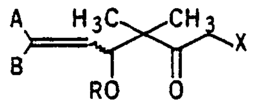

(I),

in der

A Wasserstoff, Halogen oder $C_1$- bis $C_4$-Halogenalkyl
B $C_1$- bis $C_4$-Halogenalkyl
X Wasserstoff oder Halogen und
R Wasserstoff, eine Arylsulfonyl-, Alkylsulfonyl- oder Halogenalkylsulfonylgruppe

bedeuten, Verfahren zu ihrer Herstellung und ihre Verwendung zur Herstellung von Pyrethroiden oder Pyrethroidvorprodukten der Formel (V)

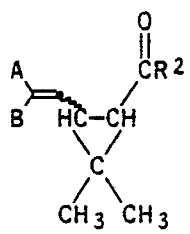

(V),

in der A, B und $R^2$ die in Anspruch 9 genannte Bedeutung haben.

Aus dem Stand der Technik, z.B. aus EP-A-95 047 oder DE-A-34 41 370, ist bekannt, daß 3-vinylsubstituierte 2,2-Dimethylcyclopropancarbonsäuren ausgehend von Verbindungen des Typs (A)

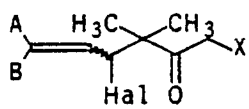

(A),

wobei A und B u.a. für Halogen oder Halogenalkyl stehen, X Wasserstoff oder Halogen und Hal Chlor oder Brom bedeuten, hergestellt werden können. Nachteilig an diesem Syntheseweg ist jedoch die Hydrolyseempfindlichkeit der Verbindungen (A) sowie ihre z.T. schwierige Zugänglichkeit.

In Synthesis 1981, 567-570 werden $\beta$-Hydroxyketone des Typs (B)

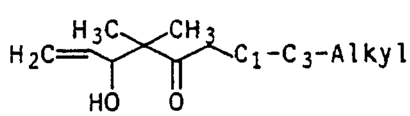

(B)

beschrieben. Ihre Bereitstellung durch Aldolkondensation bei Temperaturen von -78°C in Gegenwart von Lithiumdiisopropylamin ist jedoch insbesondere für Reaktionen im technischen Maßstab problematisch.

Der Erfindung lag nun die Aufgabe zugrunde, Verbindungen zur Verfügung zu stellen, die sich als hydrolyseunempfindliche Ausgangsstoffe zur Herstellung von Pyrethroiden der Formel (V) vorteilhaft verwenden lassen und die auf einfache und kostengünstige Weise zugänglich sind.

Demgemäß wurden die eingangs definierten neuen 3,3-Dimethyl-hex-5-en-2-on-derivate der Formel (I) und Verfahren zu ihrer Herstellung gefunden.

Im einzelnen haben die Substituenten in Formel (I) folgende Bedeutung:

A - Wasserstoff; Halogen wie Fluor, Chlor, Brom oder Jod, insbesondere Fluor, Chlor und Brom;
- $C_1$-$C_4$-Halogenalkyl, bevorzugt $C_1$-$C_4$-Fluor- und Chlorhalogenalkyl, besonders bevorzugt $C_1$-$C_4$-Perhal-

ogenalkyl wie C $Hal_3$, $C_2$ $Hal_5$, $C_3$ $Hal_7$, $C_4$ $Hal_9$, wobei Hal unabhängig voneinander Chlor oder Fluor bedeuten. Beispielsweise seien Trifluormethyl, Trichlormethyl, Dichlorfluormethyl, Difluorchlormethyl, Pentachlorethyl, Pentafluorethyl und Perchlorbutyl genannt.

B - $C_1$-$C_4$-Halogenalkyl, bevorzugt $C_1$-$C_4$-Fluor- und Chlorhalogenalkyl, besonders bevorzugt $C_1$-$C_4$-Perhalogenalkyl wie C $Hal_3$, $C_2$ $Hal_5$, $C_3$ $Hal_7$, $C_4$ $Hal_9$, wobei Hal unabhängig voneinander Chlor oder Fluor bedeuten. Beispielsweise seien Trifluormethyl, Trichlormethyl, Dichlorfluormethyl, Difluorchlormethyl, Pentachlorethyl, Pentafluorethyl und Perchlorbutyl genannt.

X - Wasserstoff oder Halogen wie Fluor, Chlor, Brom oder Jod, bevorzugt Chlor und Brom.

R - Wasserstoff oder eine Aryl-, Alkyl- oder Halogenalkylsulfonylgruppe $R'$-$SO_2$-, worin $R'$ für einen Arylrest, z.B. einen Phenyl- oder durch inerte Gruppen substituierten Phenylrest wie Halogen, Cyano, $C_1$-$C_4$-Alkyl- oder $C_1$-$C_4$-Alkoxy-substituierten Phenylrest, beispielsweise einen Tolyl-, p-Chlorphenyl- oder o-Bromphenylrest steht oder $R'$ einen verzweigten oder unverzweigten Alkylrest, z.B. $C_1$-$C_{20}$-Alkyl, insbesondere $C_1$-$C_5$-Alkyl wie Methyl, Ethyl, Propyl, iso-Propyl, Butyl, iso-Butyl, tert.-Butyl, Pentyl oder iso-Pentyl, besonders bevorzugt Methyl und Ethyl bedeutet oder $R'$ für einen verzweigten oder unverzweigten $C_1$-$C_{20}$-Halogenalkylrest steht, insbesondere $C_1$-$C_5$-Halogenalkyl, beispielsweise $C_1$-$C_5$-Fluor- oder Chloralkyl, besonders bevorzugt Trifluormethyl.

Die erfindungsgemäßen Verbindungen der Formel (I) sind lagerstabile, destillierbare und vor allem im Gegensatz zu Verbindungen der Struktur (A) hydrolysestabile Verbindungen.

Zur Herstellung der 3,3-Dimethyl-hex-5-en-2-onderivate der Formel (I) sind folgende Verfahren gefunden worden:

Im Fall von R und X gleich Wasserstoff (Formel (Ia) gemäß Anspruch 2) setzt man vorteilhaft ein 1,3-Dihalogenpropoxyderivat (II) mit Methylisopropylketon (III) in Gegenwart einer Brönstedt-Säure und mindestens einem Äquivalent Wasser, bezogen auf (II), gemäß folgendem Reaktionsschema um:

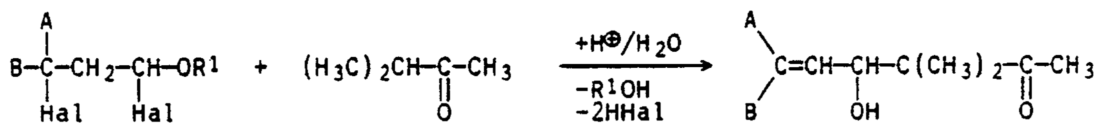


(II) (III) (Ia)

Das Gelingen dieses Verfahrens ist insbesondere im Hinblick auf das in der DE-A-34 41 370 beschriebene Verfahren zur Herstellung von trihalogen-substituierten 3,3-Dimethyl-hex-5-en-2-onverbindungen überraschend. Anstelle der erfindungsgemäßen Verbindungen (Ia) hätte man vielmehr halogensubstituierte Produkte oder Produktgemische erwartet. Die hohe Selektivität der erfindungsgemäßen Umsetzung, verbunden mit hoher Ausbeute an Hydroxyketon (Ia), war daher unvorhersehbar.

Die Hydrolysestabilität der Verbindung (Ia) ist von Vorteil, da hierdurch die Reaktionsdauer der Umsetzung so gewählt werden kann, daß der alpha-Halogenether (II) im Gemisch nicht mehr nachweisbar ist und trotzdem (I) unter den Reaktionsbedingungen nicht zerfällt.

Zur Durchführung der Umsetzung können die Komponenten (II), (III), die Brönstedt-Säure HA und Wasser in beliebiger Reihenfolge gemischt und vorteilhaft bei Temperaturen zwischen 10 und 100 °C, bevorzugt 30 bis 70 °C, zur Reaktion gebracht werden.

Um einen isothermen und gut kontrollierbaren Reaktionsverlauf zu erreichen verfährt man insbesondere bei Ansätzen im Mol-Bereich und darüber bevorzugt so, daß das Keton (III), die Saüre HA und Wasser vorgelegt und auf Reaktionstemperatur gebracht werden und anschließend das 1,3-Di-halogenpropoxyderivat (II) zugesetzt wird.

Der Substituent $R^1$ in Formel (II) bedeutet unverzweigtes oder verzweigtes $C_1$-$C_{20}$-Alkyl, vorzugsweise $C_1$-$C_8$-Alkyl, besonders bevorzugt $C_1$-$C_4$-Alkyl, wie Methyl, Ethyl, Propyl, Isopropyl, n-Butyl, iso-Butyl, sek.-Butyl oder tert.-Butyl; Aryl, vorzugsweise Phenyl, 1-Naphthyl, 2-Naphthyl, $C_7$-$C_{20}$-Aralkyl, z.B. Phenylalkyl, wie Benzyl, Phenethyl, Phenyl-n-propyl. Die genannten Reste $R^1$ können noch unter den Reaktionsbedingungen inerte Substituenten z.B. $C_1$-$C_4$-Alkylreste tragen.

Die Verbindungen (II) sind zum Teil bekannt aus EP-A-31 041 und lassen sich aus den entsprechenden Vinylethern (VI) und den Halogenkohlenwasserstoffen (VII) in hohen Ausbeuten gemäß folgender Reaktionsgleichung erhalten:

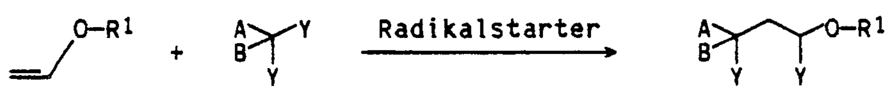


(VI) (VII) Y = Chlor, Brom (II)

indem man in Gegenwart eines an sich üblichen Radikalstarters oder durch Bestrahlung bei Temperaturen von 0 bis 120 °C, vorzugsweise von 10 bis 70 °C, gegebenenfalls in Gegenwart eines inerten Lösungsmittels umsetzt. Als Lösungsmittel sind beispielsweise die Halogenkohlenwasserstoffe (VII) geeignet.

Die Verbindungen (II) können ohne Reinigung oder Abdestillieren des Lösungsmittels zur Umsetzung mit dem Methylisopropylketon (III) eingesetzt werden. Dies ist eine besonders bevorzugte Ausführungsform des erfindungsgemäßen Verfahrens, da hierdurch die Handhabung mit dem alpha-Halogen-ether (II) auf ein Minimum begrenzt werden kann.

Es ist natürlich auch möglich (II) in reiner Form oder gelöst in einem inerten Lösungsmittel, z.B. einem inerten Halogenwasserstoff, vorzugsweise dem Halogenwasserstoff der für die Synthese von (II) verwendet wurde, für die nachfolgende Umsetzung einzusetzen.

Der Zusatz eines Lösungsmittels zur Durchführung der Umsetzung von (II) mit (III) ist nicht unbedingt erforderlich, doch können unter den Reaktionsbedingungen inerte Lösungsmittel z.B. Halogenkohlenwasserstoffe wie Tetrachlormethan, Methylenchlorid, Trifluortrichlorethan, Chloroform, Pentafluortrichlorpropan oder Kohlenwasserstoffe wie Benzol, Toluol, Xylol, Pentan oder Hexan zugesetzt werden.

Als saure Verbindungen HA eignen sich anorganische Säuren wie Salzsäure, Bromwasserstoffsäure oder Schwefelsäure bevorzugt in wäßriger Lösung, doch können die Säuren auch in ihrer wasserfreien Form eingesetzt werden.

Außerdem eignen sich auch organische Säuren HA, beispielsweise Carbonsäuren wie Ameisensäure, Essigsäure, Propionsäure, Dicarbonsäuren wie Oxalsäure oder Sulfonsäuren wie Methansulfonsäure, Benzolsulfonsäure, p-Toluolsulfonsäure. Weiterhin geeignet sind saure Ionenaustauschharze.

Die Menge an Säure HA ist nicht kritisch, da die Säure bei der Reaktion nicht verbraucht wird, sondern im Gegenteil zwei Äquivalente Halogenwasserstoff bei der Umsetzung entstehen. Um längere Induktionsperioden zu vermeiden, setzt man bevorzugt mindestens 10 Mol% bis 10 Moläquivalente Säure HA, bezogen auf (II), zu. Größere Überschüsse sind möglich, aber wenig wirtschaftlich.

Die Menge an Wasser soll mindestens ein Mol betragen, doch verwendet man bevorzugt größere Überschüsse, um die entstehende Halogenwasserstoffsäure in der Reaktionsmischung zu absorbieren. Im allgemeinen können Mengen von 1 bis 100 mol Wasser, insbesondere 2 bis 50 mol, bezogen auf (II), verwendet werden.

(II) und (III) werden in der Regel in äquimolarem Verhältnis eingesetzt, doch kann (III) auch im Überschuß verwendet werden. Ein Überschuß von (II) ist aufgrund von Nebenproduktbildung und wegen der Toxizität der alpha-Halogenether (II) wenig sinnvoll. Im Überschuß eingesetztes (III) kann nach der Reaktion durch Destillation sehr einfach vom Wertprodukt abgetrennt werden.

Die β-Hydroxyketone (I) mit R = H lassen sich durch Umsetzung mit Sulfonsäurehalogeniden der Formel (IV)

$R'-SO_2-Hal$ (IV),

worin R′ die obengenannte Bedeutung hat und Hal für Halogen wie Fluor, Chlor oder Brom, vorzugsweise Chlor steht, in an sich bekannter Weise in Gegenwart einer Base in β-Hydroxyketone (I) mit R = Aryl-, Alkyl- oder Halogenalkylsulfonyl überführen.

Als Base können an sich übliche Mittel, die nicht zur Verseifung des Sulfonsäurehalogenids führen, verwendet werden. Vorzugsweise werden tertiäre Amine, insbesondere $C_1$-$C_8$-Trialkylamine oder basische Heteroaromaten wie Pyridin oder alkylsubstituierte Pyridine eingesetzt.

Beispielsweise seien folgende Amine aufgeführt: Trimethyl-, Triethyl-, Tri-n-propyl-, N,N-Dimethylethyl-, N,N-Dimethylisopropyl-, Tri-n-butylamin, Pyridin oder substituierte Pyridine wie 2,3- oder 4-Methylpyridin oder Dimethylpyridine wie 2,6-Dimethylpyridin.

Die Menge an Base beträgt mindestens ein Moläquivalent, bezogen auf (I), vorzugsweise kann man einen geringen Überschuß, z.B. 1,05 bis 1,1 mol Base pro Mol (I), einsetzen.

Von Vorteil ist der Zusatz katalytischer Mengen, z.B. 0,01 bis 1 Mol%, eines Acylierungskatalysators wie 4-(N,N-Dimethylamino)-pyridin.

Die Umsetzung kann in Gegenwart oder Abwesenheit eines Lösungsmittels durchgeführt werden. Als Lösungsmittel kommen inerte organische Lösungsmittel wie Kohlenwasserstoffe wie Benzol, Toluol, Petrolether, chlorierte Kohlenwasserstoffe wie Methylenchlorid, Chloroform, Tetrachlorkohlenstoff, Ether wie Dioxan, THF oder die Base selbst in Frage.

Die Verbindungen (I) mit X = Halogen erhält man vorteilhaft, indem man die Verbindungen (I) mit X = H mit einem Halogenierungsmittel, gegebenenfalls in einem inerten Lösungsmittel umsetzt. Als Halogenisierungsmittel kommen Chlor, Brom, Sulfurylchlorid, Sulfurylbromid oder N-Halogenverbindungen wie N-Bromsuccinimid (NBS) oder N-Chlorsuccinimid (NCS) in Frage.

Die Umsetzung wird üblicherweise in einem inerten Verdünnungsmittel durchgeführt. Als solche dienen beispielsweise Chlorkohlenwasserstoffe wie Methylenchlorid, Chloroform, Tetrachlorkohlenstoff, Dichlorethan oder hochchlorierte Aromaten, ferner Carbonsäuren wie beispielsweise Essigsäure oder aliphatische Kohlenwasserstoffe, z.B. Petrolether.

Gegebenenfalls kann die Halogenierung in Gegenwart eines Katalysators, z.B. Halogenwasserstoffsäuren wie Chlorwasserstoff oder Bromwasserstoff oder Lewis-Säuren wie Aluminiumchlorid, Zinkchlorid oder Aluminiumbromid vorgenommen werden.

Die Reaktionstemperatur sollte +70 °C nicht überschreiten, vorzugsweise wird die Umsetzung bei Temperaturen von -10 und +35 °C durchgeführt.

In der Regel wird maximal bis zu einem Äquivalent Halogenierungsmittel zugegeben; es kann jedoch auch ein Unterschuß verwendet werden, um zu verhindern, daß eine weitere Halogenierung des Moleküls eintritt. Vorteilhaft sind Mengen von 0,8 bis 1 mol Halogenierungsmittel.

Die erfindungsgemäßen Verbindungen (I), in denen X für Halogen und der Rest R für eine Aryl-, Alkyl- oder Halogenalkylsulfonylgruppe steht, können unter basischen Bedingungen in die 2,2-Dimethylcyclopropan-1-carbonsäure und ihre Derivate überführt werden.

Es wurde gefunden, daß es von den bei der Cyclisierung verwendeten basischen Bedingungen abhängt, ob man die freie Carbonsäure erhält oder einen beliebigen Ester.

Die freie Carbonsäure erhält man bei der Verwendung von Alkali- oder Erdalkalihydroxiden wie Natriumhydroxid, Kaliumhydroxid, Lithiumhydroxid, Calciumhydroxid oder Bariumhydroxid, wobei aus Kostengründen Kalium- oder Natriumhydroxid bevorzugt sind.

Verwendet man jedoch Alkoholate oder auch Phenolate oder Alkohole und Phenole in Gegenwart starker Basen wie Natriumhydrid oder Kalium-tert.-butylat, so erhält man die 2,2-Dimethyl-1-carbonsäureester (V) dieser Alkohole bzw. Phenole.

Als Alkohole eignen sich:

1. Alkohole mit verzweigten oder unverzweigten $C_1$-$C_8$-Kohlenstoffketten, z.B. Methanol oder Ethanol.

2. Alkohole der Formel (B),

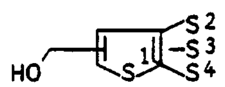

(B)

in der

$S^1$ einen zweiwertigen Sauerstoffrest, zweiwertigen Schwefelrest oder eine Vinylengruppe,

$S^2$, $S^3$ und $S^4$ unabhängig voneinander einen Rest aus der Gruppe Wasserstoff, $C_1$-$C_4$-Alkyl, Halogen, $C_1$-$C_4$-Alkoxy, Halogenalkyl, Halogenalkoxy, $C_1$-$C_4$-Alkylen, Phenyl, Phenoxy, Benzyl und Phenylthio bedeuten oder jeweils zwei Reste aus der Gruppe $S^2$, $S^3$ und $S^4$ verbunden sind und eine zweiwertige Methylendioxygruppe bilden, die mit zwei benachbarten Ringkohlenstoffatomen eines Phenylrings verknüpft ist und sofern die Reste $S^2$, $S^3$ oder $S^4$ einen Phenylring enthalten, so kann dieser mit 1 bis 3 Substituenten aus der Gruppe Halogen, $C_1$-$C_4$-Alkoxy und $C_1$-$C_4$-Alkyl substituiert sein.

3. Alkohole der allgemeinen Formel (C),

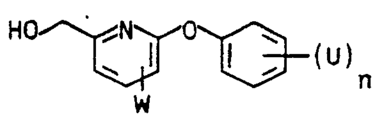

(C),

in der U Wasserstoff, $C_1$-$C_4$-Alkyl, $C_1$-$C_4$-Alkoxy, $C_1$-$C_4$-Alkylthio, $C_1$-$C_4$-Alkylsulfonyl, Trifluormethyl, 3,4-Methylendioxy, Chlor, Fluor oder Brom bedeutet, n gleich 1 oder 2 ist, und W die Bedeutung Wasserstoff, Chlor oder Fluor hat.

4. Alkohole der Formel (D),

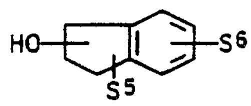

(D),

wobei $S^5$ ein Wasserstoffatom, eine $C_1$-$C_4$-Alkyl-, $C_1$-$C_4$-Alkoxy-, $C_1$-$C_4$-Alkenyl-, $C_1$-$C_4$-Alkenyloxy-, Phenyl- oder Benzylgruppe bedeutet und $S^6$ Wasserstoff, Halogen, $C_1$-$C_4$-Alkyl, $C_1$-$C_4$-Alkoxy, $C_1$-$C_4$-Alkenyl, $C_1$-$C_4$-Alkenyloxy, $C_1$-$C_4$-Alkylthio, Phenyl, Phenoxy oder Benzoyl oder eine Nitrogruppe oder Cyanogruppe darstellt.

5. Alkohole der Formel (E),

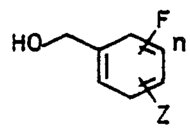

(E),

in der Z die Bedeutung Wasserstoff, Halogen, $C_1$-$C_4$-Alkyl, $C_1$-$C_4$-Alkenyl, Benzyl, $C_1$-$C_4$-Alkoxy, $C_1$-$C_4$-Alkenyloxy, $C_1$-$C_4$-Alkylthio, Alkylsulfonyl, Alkylsulfinyl, Trifluormethyl, Amino, Alkoxymethyl, Alkylthiomethyl, Phenoxy, Dialkylaminomethyl, Dialkylamino, Monoalkylamino, Aryloxyalkyl oder Alkenyloxyalkyl hat und n gleich 3 oder 4 ist.

Bevorzugt, besonders im Hinblick auf die Synthese biologisch wirksamer Pyrethroide der Formel (IV), sind die Alkoholtypen 2 bis 5.

Typische Vertreter dieser Alkohole sind z.B.:

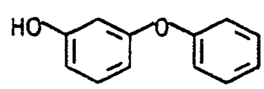

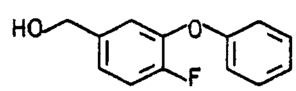

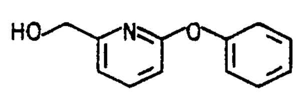

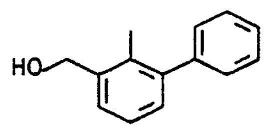

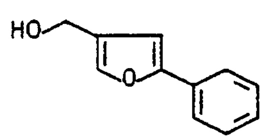

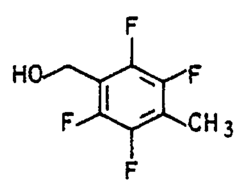

Als Phenole eignen sich z.B. Verbindungen der Struktur (F)

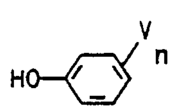

(F),

wobei V ein beliebiger unter den basischen Reaktionsbedingungen stabiler Rest ist und n eine Zahl von 0 bis 5 darstellt.

Als bevorzugte Reste V können unabhängig voneinander stehen: Halogene wie Chlor, Fluor, $C_1$-$C_4$-Alkylgruppen wie Methyl, Ethyl, Propyl, Butyl, $C_1$-$C_4$-Alkoxygruppen wie Methoxy, Ethoxy, Propoxy, Butoxy oder Phenoxygruppen, bevorzugt p oder m-Phenoxy.

Bevorzugt wird bei Verwendung der Hydroxide in Wasser und/oder einem inerten Verdünnungsmittel gearbeitet. Hier kommen beispielsweise Alkohole wie Methanol, Ethanol, tert.-Butanol, Ether wie Dioxan, Tetrahydrofuran (THF), Dimethoxyethan oder Ketone wie Aceton sowie Dimethylformamid in Frage. Es können jedoch auch nicht mit Wasser mischbare Lösungsmittel wie Methylenchlorid, Petrolether, Cyclohexan, Toluol oder Chlorbenzol, ggf. in Gegenwart eines Phasentransferkatalysators, verwendet werden.

Bei Verwendung der Alkoholate wird die Umsetzung am günstigsten in den entsprechenden Alkoholen oder inerten Lösungsmitteln wie Ethern, z.B. Dioxan oder THF durchgeführt.

Pro Mol Ausgangsstoff der Formel (I) werden mindestens 2 Äquivalente des Hydroxids bzw. Alkoholats oder Phenolats eingesetzt. Ein Überschuß an Base bis zu 3 Äquivalenten ist meist vorteilhaft. Im allgemeinen können 2 bis 5 mol pro Mol (I) eingesetzt werden, ein größerer Überschuß ist möglich, bringt aber keine weiteren Vorteile.

Die Reaktionstemperaturen können in einem größeren Bereich variiert werden, jedoch gelingt die Reaktion überraschenderweise bereits unter äußerst schonenden Bedingungen. Im allgemeinen liegen die Temperaturen zwischen 0 und 150° C, vorzugsweise zwischen 30 und 70° C.

Die Aufarbeitung des Reaktionsgemisches erfolgt bei der Herstellung der Säuren vorteilhaft durch Extraktion im Alkalischen und nach Ansäuern der Wasserphase durch erneute Extraktion. Bei Herstellung von Estern kann die Reinigung durch Destillation erfolgen. Vorher wird das Reaktionsgemisch mit Wasser verdünnt, neutral gestellt und extrahiert.

Unter den angegebenen Bedingungen enstehen cis/trans-Gemische der Säuren bzw. der Ester (V) in etwa im Verhältnis 25:75 bis 35:65.

Die neuen 3,3-Dimethyl-hex-5-en-2-onderivate der Formel (I) sind wertvolle Zwischenprodukte für die Synthese von Pflanzenschutzmitteln, insbesondere von Insektiziden, z.B. Pyrethroiden wie sie z.B. in den Patenten US-A-4,332,815, US-A-4,235,927, EP-B-3 336 bzw. Anmeldungen GB-A-2 000 764, EP-A-31 199, EP-A-200 943, EP 145 179, die Pyrethroide mit $CF_3$/Cl-Säurekomponente enthalten, genannt sind.

Die nachfolgenden Beispiele sollen die Erfindung weiter erläutern. Die angegebenen Produktreinheiten wurden gaschromatographisch ermittelt.

A Herstellung der Ausgangsstoffe (II):

Beispiel 1

1-(n-Butoxy)-1,3,3-trichlor-4,4,4-trifluorpentan

2,06 kg 1,1,1-Trichlor-2,2,2-trifluorethan und 9,16 g Azoisobutyronitril wurden unter Rückflußtemperatur mit 458 g n-Butyl-vinylether versetzt. Die Mischung wurde noch 6 h zum Rückfluß erhitzt und anschließend nicht umgesetztes Perhalogenalkan im Vakuum abdestilliert. Als Rückstand erhielt man 1,05 kg Produkt.
Ausbeute: 80 % d. Th.

B. Herstellung der erfindungsgemäßen Verbindungen (I):

Beispiel 2

6-Chlor-3,3-dimethyl-4-hydroxy-7,7,7-trifluor-hept-5-en-2-on

759 g (2,6 mol) 1-(n-Butoxy)-1,3,3-trichlor-4,4,4-trifluorpropan und 681 g (7,9 mol) Methyl-isopropylketon wurden bei 55° C innerhalb von 2 h mit 200 ml konzentrierter Salzsäure versetzt. Die Mischung wurde noch 30 min bei 55° C nachgerührt, anschließend mit Wasser verdünnt und die organische Phase abgetrennt. Nach dem Abdestillieren von nicht umgesetztem Methyl-isopropylketon und anschließender Destillation im Vakuum erhielt man 580 g Produkt.
Ausbeute: 90 % d. Th.
Reinheit: 96,7 %

Beispiel 3

6-Chlor-3,3-dimethyl-4-methansulfonyloxy-7,7,7-trifluor-hept-5-en-2-on

733 g (3 mol) 6-Chlor-3,3-dimethyl-4-hydroxy-7,7,7-trifluor-hept-5-en-2-on, 360 g (3,6 mol) Triethylamin und 1,5 g 4-(N,N-Dimethylamino)-pyridin in 1 l Toluol wurden bei 5 °C mit 378 g (3,6 mol) Methansulfonsäurechlorid versetzt. Die Mischung wurde 3 h bei 25 °C nachgerührt, der Niederschlag abfiltriert und das Filtrat mit verdünnter Salzsäure und mit gesättigter Natriumhydrogencarbonatlösung extrahiert. Nach dem Trocknen und Einengen der organischen Phase erhielt man 948 g Produkt. Fp.: 50-52 °C.
Ausbeute: 97,9 % d.Th.
Produktreinheit: 98,7 %

Beispiel 4

1-Brom-6-chlor-3,3-dimethyl-4-methansulfonyloxy-7,7,7-trifluor-hept-5-en-2-on

720 g (2,2 mol) 6-Chlor-3,3-dimethyl-4-methansulfonyloxy-7,7,7-trifluor-hept-5-en-2-on in 2 l Chloroform wurden bei 20 °C mit 360 g (2,2 mol) Brom versetzt. Die Mischung wurde 2 h bei 20 °C nachgerührt und anschließend eingeengt. Man erhält 781 g Produkt.
Ausbeute: 87,2 % d.Th.
Produktreinheit: 82 %, neben 2,3 % 1,1-Dibrom-6-chlor-3,3-dimethyl-4-methansulfonyloxy-7,7,7-trifluor-hept-5-en-on und 15,7 % nicht umgesetztem Ausgangsmaterial.
Das Ausgangsmaterial kann, wie in Beispiel 6 angegeben, zurückgewonnen und erneut zur Bromierung eingesetzt werden.

Beispiel 5

Synthese von (Ia) ohne Isolierung des alpha-Halogenethers

1687 g (9 mol) 1,1,1-Trichlor-2,2,2-trifluorethan und 750 g (7,5 mol) Isobutyl-vinylether wurden mit 7 g Azobisisobutyronitril versetzt und 4 h zum Rückfluß erhitzt. Die Mischung enthielt nach gaschromatographischer Analyse 66 % an alpha-Chlorether.

Diese Mischung wurde ohne weitere Reinigung zu einer auf 60 °C erwärmten Lösung, bestehend aus 1,9 kg (22 mol) Methylisopropylketon und 3 l halbkonzentrierter Salzsäure, gegeben und bei 60 °C nachgerührt, bis gaschromatographisch kein alpha-Chlorether mehr nachweisbar war. Der Reaktionsaustrag wurde mit Wasser verdünnt, die organische Phase abgetrennt, getrocknet und destilliert. Der Vorlauf enthielt nicht umgesetztes Trichlortrifluorethan und Methylisopropylketon, sowie das Reaktionsprodukt iso-Butanol. Die Komponenten können durch Feindestillation getrennt werden.
Die Hauptfraktion besteht aus 1,19 kg Produkt.
Ausbeute: 65 % d.Th., bezogen auf den Vinylether
Produktreinheit: 98,7 %.

C Herstellung der 2,2-Dimethylcyclopropyl-1-carbonsäuren und deren Ester

Beispiel 6

3-(2-Chlor-3,3,3-trifluorprop-1-en-1-yl)-2,2-dimethylcyclopropan-carbonsäure

Eine nach Beispiel 4 erhaltene Mischung bestehend aus 602 g (1,5 mol) 1-Brom-6-chlor-3,3-dimethyl-4-methansulfonyloxy-7,7,7-trifluor-hept-5-en-2-on und 115 g (0,35 mol) 6-Chlor-3,3-dimethyl-4-methansulfonyloxy-7,7,7-trifluor-hept-5-en-2-on, wurde in 1,2 l 10 %iger wäßriger Natronlauge bei 70 bis 80 °C ca. 15 min gerührt bis eine klare Lösung entstanden war.

Die Mischung wurde abgekühlt und mit Methylenchlorid extrahiert. Aus dem Extrakt erhielt man nach

dem Trocknen und Einengen 105 g 6-Chlor-3,3-dimethyl-4-methansulfonyl-7,7,7-trifluor-hept-5-en-2-on zurück, welches nach Beispiel 4 erneut zur Bromierung eingesetzt werden kann.

Die alkalische Wasserphase wurde mit Salzsäure sauer gestellt und das ausgefallene Produkt durch Extraktion mit Methylenchlorid gewonnen. Man erhielt 341 g einer kristallinen Masse, bestehend aus 92 g cis-3-(Z-2-Chlor-3,3,3-trifluorprop-1-en-1-yl)-2,2-dimethylcyclopropan-carbonsäure und 206 g trans-3-(Z-2-Chloro-3,3,3-trifluorprop-1-en-1-yl)-2,2-dimethylcyclopropan-carbonsäure.
Ausbeute: 82 % d. Th.

Beispiel 7

3-(2-Chlor-3,3,3-trifluorprop-1-en-1-yl)-2,2-dimethylcyclopropan-carbonsäure-3-phenoxybenzylester

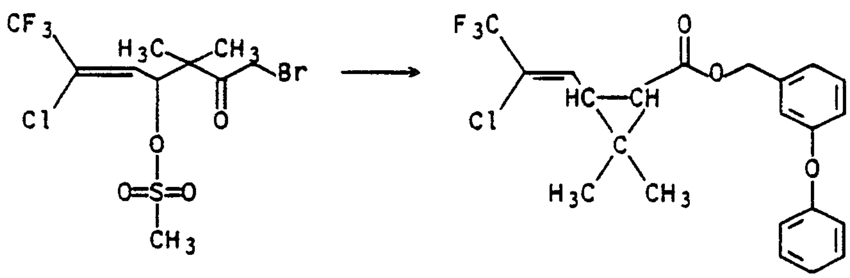

Zu 7,2 g (0,3 mol) Natriumhydrid in 50 ml Dioxan tropfte man bei 80 °C 66 g (0,3 mol) 3-Phenoxybenzylalkohol, gelöst in 50 ml Dioxan. Die Mischung wurde bis zur Beendigung der Gasentwicklung bei 80 °C gerührt. Anschließend kühlte man auf 25 °C ab und tropfte eine Lösung aus 40,2 g (0,1 mol) 1-Brom-6-chlor-3,3-dimethyl-4-methansulfonyloxy-7,7,7-trifluorhept-5-en-2-on gelöst in 50 ml Dioxan zu. Nach 2 h bei Raumtemperatur goß man die Mischung in Wasser und extrahierte mit Methylenchlorid. Der überschüssige Alkohol wurde durch Destillation im Vakuum oder durch Chromatographie an Kieselgel vom Produkt abgetrennt. Man erhielt 40 g Phenoxybenzylalkohol zurück und 24,3 g 3-(2-Chlor-3,3,3-trifluorprop-1-en-1-yl)-2,2-dimethylcyclopropan-carbonsäure-3′-phenoxybenzylester.
Ausbeute: 57 % d. Th.

Beispiel 8

3-(2-Chlor-3,3,3-trifluorprop-1-en-1-yl)-2-dimethylcyclopropan-carbonsäuremethylester

Zu 40 g (0,1 mol) 1-Brom-6-chlor-3,3-dimethyl-4-methansulfonyloxy-7,7,7-trifluor-hept-5-en-2-on in 100 ml Methanol tropfte man 90 g einer 30 %igen methanolischen Natriummethylatlösung. Die Mischung wurde auf 50 °C erwärmt, bis gaschromatographisch kein Ausgangsprodukt mehr nachweisbar war (ca. 4 h). Anschließend goß man die Mischung in Wasser, stellte mit 1n Salzsäure auf pH 7 und extrahierte mit Methylenchlorid. Nach der Destillation des Rückstandes erhielt man 18,4 g Produkt.
Ausbeute: 72 % d.Th.

## Ansprüche

1. 3,3-Dimethyl-hex-5-en-2-onderivate der allgemeinen Formel (I)

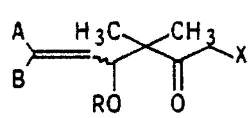

(I)

in der
A Wasserstoff, Halogen oder $C_1$- bis $C_4$-Halogenalkyl
B $C_1$- bis $C_4$-Halogenalkyl
X Wasserstoff oder Halogen und
R Wasserstoff, eine Arylsulfonyl-, Alkylsulfonyl- oder Halogenalkylsulfonylgruppe
bedeuten.

2. Verfahren zur Herstellung von 3,3-Dimethyl-hex-5-en-2-onderivaten der Formel (Ia)

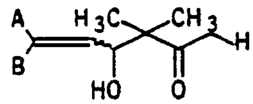

(Ia)

in der
A für Wasserstoff, Halogen oder $C_1$- bis $C_4$-Halogenalkyl und B für $C_1$- bis $C_4$-Halogenalkyl stehen, dadurch gekennzeichnet, daß man 1,3-Dihalogenpropoxyderivate der Formel (II)

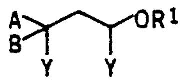

(II)

in der
A,B die obengenannte Bedeutung haben
Y für Brom oder Chlor und
$R^1$ für $C_1$- bis $C_{20}$-Alkyl, Aryl oder $C_7$- bis $C_{20}$-Aralkyl stehen,
mit Isopropylmethylketon (III) in Gegenwart einer Brönstedt-Säure und mindestens äquimolaren Mengen Wasser, bezogen auf (II), umsetzt.

3. Verfahren nach Anspruch 2, dadurch gekennzeichnet, daß man als Brönstedt-Säuren Mineralsäuren, Carbonsäuren, Sulfonsäuren oder saure Ionenaustauscher verwendet.

4. Verfahren nach Anspruch 2, dadurch gekennzeichnet, daß man 10 mol.% bis 10 mol Säure pro Mol (II) verwendet.

5. Verfahren zur Herstellung von 3,3-Dimethyl-hex-5-en-2-onderivaten der Formel (I)

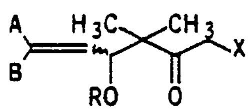

(I),

in der A, B und X die in Anspruch 1 genannte Bedeutung haben und R eine Arylsulfonyl-, Alkylsulfonyl- oder Halogenalkylsulfonylgruppe darstellt, dadurch gekennzeichnet, daß man 3,3-Dimethyl-hex-5-en-2-onderivate (I) mit R = H in Gegenwart einer Base mit einem Sulfonsäurehalogenid der Formel (IV)
$R'-SO_2-Hal$ (IV),
in der
Hal für Fluor, Chlor oder Brom und
$R'$ für Alkyl, Aryl oder Halogenalkyl steht,
umsetzt.

6. Verfahren nach Anspruch 5, dadurch gekennzeichnet, daß man tertiäre Amine oder Pyridine als Base verwendet.

7. Verfahren zur Herstellung von 3,3-Dimethyl-hex-5-en-2-onderivaten der Formel (I)

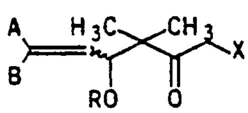

(I),

in der A, B und R die in Anspruch 1 genannte Bedeutung haben und X Halogen darstellt, dadurch gekennzeichnet, daß man 3,3-Dimethyl-hex-5-en-2-onderivate (I) mit X = H in an sich bekannter Weise mit

einem Halogenierungsmittel umsetzt.

8. Verfahren nach Anspruch 7, dadurch gekennzeichnet, daß man als Halogenierungsmittel Chlor, Brom, Sulfurylchlorid, N-Brom- oder N-Chlor-succinimid verwendet.

9. Verfahren zur Herstellung von Pyrethroiden oder Pyrethroidvorprodukten der Formel (V)

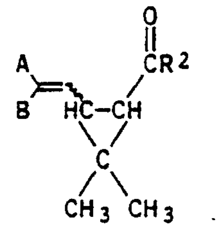

(V)

in der A und B die in Anspruch 1 genannte Bedeutung haben und $R^2$ einen Hydroxyl-, Alkoxy- oder Phenoxyrest oder einen üblichen Pyrethroidalkoholrest bedeutet, dadurch gekennzeichnet, daß man 3,3-Dimethyl-hex-5-en-2-onderivate der Formel (I)

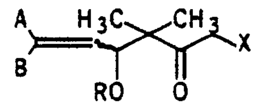

(I),

in der

X Halogen und

R Arylsulfonyl, Alkylsulfonyl oder Halogenalkylsulfonyl bedeuten,

mit mindestens 2 Äquivalenten eines Alkali- oder Erdalkalihydroxids im Fall von $R^2$ = OH oder eines Alkali- oder Erdalkalialkoholats eines Alkohols bzw. Phenols $R^2OH$ umsetzt.